Europäisches Patentamt

European Patent Office

Office européen des brevets

(19)

(11) Publication number: **0 031 850**
**B2**

(12) NEW EUROPEAN PATENT SPECIFICATION

(45) Date of publication of the new patent specification: **14.12.88**

(21) Application number: **80901620.7**

(22) Date of filing: **07.07.80**

(86) International application number:
**PCT/US80/00841**

(87) International publication number:
**WO 81/00209 05.02.81 Gazette 81/04**

(51) Int. Cl.⁴: **A 61 M 1/00, F 16 K 31/08**

(54) MAGNETICALLY CONTROLLED DRUG INFUSION SYSTEM.

(30) Priority: **13.07.79 US 57167**

(43) Date of publication of application:
**15.07.81 Bulletin 81/28**

(45) Publication of the grant of the patent:
**14.03.84 Bulletin 84/11**

(45) Mention of the opposition decision:
**14.12.88 Bulletin 88/50**

(84) Designated Contracting States:
**AT CH DE FR GB LI LU NL SE**

(56) References cited:
**DE-A-2 155 883**
**DE-A-2 400 392**
**FR-A-2 305 197**
**US-A-2 310 562**
**US-A-2 575 086**
**US-A-2 736 331**
**US-A-3 270 763**
**US-A-3 570 806**
**US-A-3 731 681**
**US-A-3 890 968**

(73) Proprietor: **REGENTS OF THE UNIVERSITY OF MINNESOTA**
**100 Church Street Southeast**
**Minneapolis, MN-55455 (US)**

(72) Inventor: **DORMAN, Frank D.**
**3048 Hayes N.E.**
**Minneapolis, MN 55418 (US)**

(74) Representative: **Ellis, John Clifford Holgate et al**
**MEWBURN ELLIS & CO. 2/3 Cursitor Street**
**London EC4A 1BQ (GB)**

(56) References cited:
**TRANSACTIONS OF THE AMERICAN SOCIETY FOR ARTIFICAL INTERNAL ORGANS, VOLUME 14, ISSUED 27 APRIL 1978, P.R. PERKINS ET AL, DESIGN AND INITAL TESTING OF A TOTALLY IMPLANTABLE TRANSCUTANEOUSLY CONTROLLABLE INSULIN DELIVERY DEVICE, PAGES 229-231**

EP 0031 850 B2

EP 0 031 850 B2

(56) References cited:

DIABETES, VOLUME 28, ISSUED JULY 1979, B.J. BLACKSHEAR ET AL, CONTROL OF BLOOD GLUCOSE IN EXPERIMENTAL DIABETES BY MEANS OF A TOTALLY IMPLANTABLE INSULIN INFUSION DEVICE, PAGES 634-638 CHEMICAL ENGINEER'S HANDBOOK, FIFTH EDITION, R.H. PERRY, 1973, McGRAW-HILL BOOK CO., PAGE 23-60

MEDICAL PROGRESS THROUGH TECHNOLOGY, vol. no, 6, no. 4, November 1979 Springer-Verlag P.J. BLACKSHEAR et al. "THE IMPLANTABLE INFUSION PUMP: A NEW CONCEPT IN DRUG DELIVERY", PAGES 149-161

## Description

### Technical field

The present invention relates to the field of medical devices, more particularly, an accessory for controlling a drug infusion device.

### Background of the invention

This invention is directed to an accessory device for accurately controlling the flow rate of drugs from drug delivery devices that depend on a fluid restriction to limit the flow rate from a pressurized drug storage chamber. One device of this type is the implantable infusion pump illustrated and described in U.S. Patent No. 3,731,681, the disclosure of which is incorporated herein by reference. Specifically, the present invention is directed to a magnetically controlled valving system designed to permit variable flow rates dependent upon need while guarding against over-doses of drugs. The magnetically controlled valve is used to modulate the flow rate of drug delivered from the constant pressure source in the implanted infusion pump.

When an implanted infusion pump is used, for example, to deliver insulin for the treatment of diabetes, two different flow rates are desirable, ranging from a low of about 1 cc/day to a high of from about 10 to 15 cc/day. The high flow rate of this magnitude is needed to deliver insulin fast enough to compensate for the absorption of glucose from ingested meals, the function of the insulin being to keep the glucose level in the bloodstream within prescribed limits. Administration of insulin at high levels could be fatal if continued for any substantial period of time after the glucose absorption is completed. Even the delivery of what would be a normal high dose of insulin after a meal would be harmful if for some reason that meal was not absorbed normally, due to vomitting, for example. Another hazard would arise in the event of mechanical failure of some component in the pump permitting the stored dose of insulin, which is intended to last for at least one month, to be released too rapidly for the body to use. These hazards are compounded by the fact that the periodic high flow rate needed for proper therapy is above the body tolerance if sustained for too long a period of time.

Use of the implantable drug infusion pump of U.S.—A—3,731,681 has been largely limited to situations calling for a continuous infusion rate, such as heparinization. Use for treatment of insulin, for example, has been handicapped by inability to dispense a drug, insulin in this instance, at different infusion rates. Although some efforts have been made toward providing different flow rates wholly by implanted electronic means, inherent problems remain due to unanticipated situations of use. The earliest form of the present invention is described in a paper by the present inventor and his co-workers (Perkins et al: Design and Initial Testing of a Totally Implantable Transcutaneously Controllable Insulin Delivery Device, Trans. Am. Soc. Artif. Intern. Organs. Vol. IV, pp 229—31, 1978), incorporated herein by reference.

In one prior device disclosed in FR—A—2 305 197, there is described schematically an infusion system intended to be implanted within an animal body. The valve in that disclosure is not specifically detailed and shown only schematically. A valve from a non-analogous art (refrigeration) is disclosed in U.S.—A—2 575 086, which is magnetically actuated but is not suitable for implantation. The problems of biocompatibility, and exceptional reliability, place extreme constraints on what can be manufactured with assurance that manufacturing tolerances will not result in unreliable performance which can be disastrous in this technology.

The present invention is directed to an implantable magnetically controlled system for the infusion of drugs into an animal body from a pressure actuated drug delivery device. Although the system may be electronically controlled, the electronic control may be overriden by an external magnet, or the system may be wholly controlled by an external magnet. The system includes a body of inert biocompatible nontoxic material and a movable valving element located within said body in a cavity having an inlet leading into the cavity and an outlet leading therefrom for connection to a catheter. The invention is characterised in that the cavity is a shallow cavity and the movable valving element within the cavity is positioned so as to move between an open position in which both the inlet and the outlet are free and a disclosed position in which one of the said inlet or outlet is obstructed. The valving element has associated therewith a permanent armature magnet located within the cavity, this being actuated by magnetic actuation means external to and separate from the said body. Further, the system includes within the body biasing magnet means in the form of one or a pair of permanent magnets or ferromagnetic shunt plates which biasing means is of equal or lesser field intensity compared to the strength of the armature magnet. When the valving element is in its closed position, it is held in this position by the biasing magnet means within the body, in the absence of any external magnetic field.

The valving element is preferably a flap disk secured on one side to the body and adapted to obstruct one of the openings, preferably the outlet, into the cavity. A bypass passage may be provided, preferably through the valve body and in communication with the inlet to the body cavity, to provide for a continuous low infusion rate as determined by a flow restrictor of high resistance. The valve outlet should then preferably be connected to a restrictor of lesser resistance so that when the drug is delivered through the valve, a different and higher flow rate may be achieved. The valve may be activated magnetically either by means of a strong permanent magnet outside of the body held close to the implanted valve or by an internal implanted electromagnetic coil associated with the valve body and connected to an implanted power source through an appropriate control circuit.

Brief description of the drawings

The invention is illustrated in the accompanying drawings in which corresponding parts are identified by the same numerals and in which:

Figure 1 is a schematic representation of the implantable drug delivery system according to the present invention, showing the magnetically controllable valve in vertical section;

Figure 2 is a schematic of an electronic control system for the magnetically controllable valve;

Figure 3 is a schematic representation of the physical configuration of the drug delivery system in which the electronic control unit is in a separate but connected package; and

Figure 4 is a similar schematic of the system in which the elctronic control package is mated to the pump and valve bodies.

Detailed description of the preferred embodiment

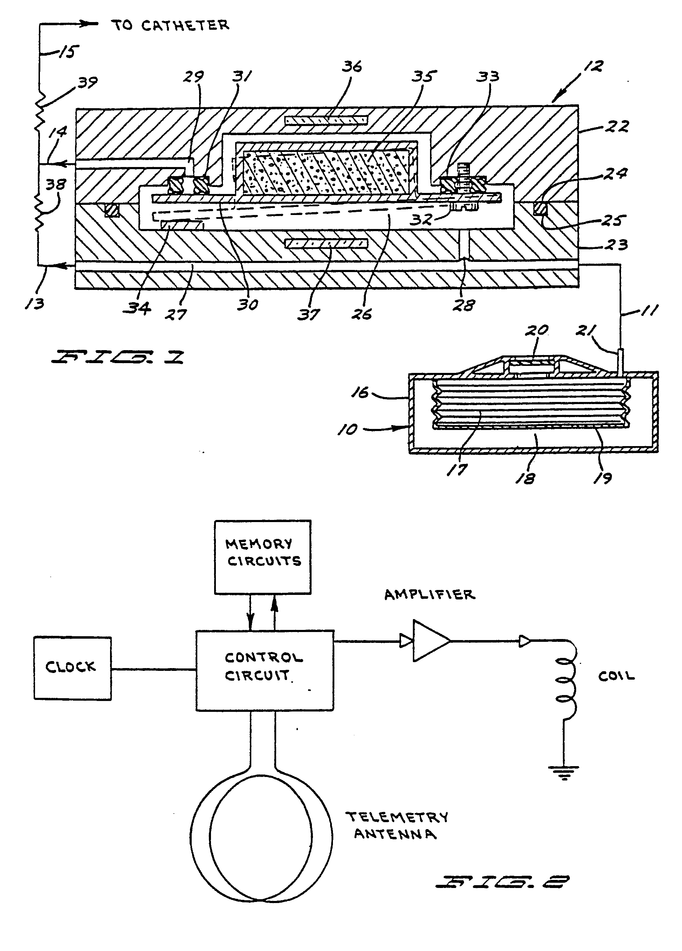

Referring now to the drawings, and particularly to Figure 1, there is shown one form of pressure actuated drug delivery system, such as an implantable infusion pump, indicated generally at 10, connected through a drug flow line 11 to a magnetically controlled valve, indicated generally at 12. Valve 12 in turn is connected through flow lines 13, 14 and 15 to a catheter, not shown, leading to the desired infusion site.

The exemplary drug delivery device, implantable infusion pump 10, comprises a housing 16 divided into a drug chamber 17 and a propellant chamber 18 by means of a bellows diaphragm 19. The infuson pump is implanted in an animal body under the skin surface so that the drug chamber may be replenished hypodermically through the skin and through a penetrable resilient stopper 20. The propellant chamber 18 contains a liquid whose vapor pressure is such that, under the conditions of normal body temperature, pressure is exerted upon the bellows to force the drug contained therein out through discharge opening 21 through the flow line 11 to the valve 12.

Valve 12 includes a body composed of mating upper and lower portions 22 and 23 fastened together as by welding, or by means of screws or equivalent fastening members. A resilient O-ring 24 is disposed in a recess 25 in the surface between the mating body members to seal the unit. The valve body encloses a shallow cavity 26, preferably circular, formed in the upper and lower body members. The valve body is preferably formed from titanium, which is inert and biocompatible. A bypass flow passage 27 preferably passing through the lower valve body member 23, connects flow lines 11 and 13. An inlet opening 28 is provided to valve cavity 26 to permit drug flow from the pump and flow line 11 through the valve. An outlet opening 29 from the valve cavity permits flow of drug from the valve to flow line 14.

A valving element in the form of a hinged disk 30 functions to open or close outlet opening 29. A resilient O-ring 31 is seated in a recess around the opening and engages the surface of valve disk 30 to insure a tight seal. Disk 30 is hinged adjacent its edge diametrically opposite from its engagement with the outlet opening, as by means of a screw 32 extending through an O-ring 33 into the cavity wall defined by the lower surface of upper valve body member 22. Valve disk 30 is preferably formed from titanium which is inert, bio-compatible, and has a long flex life. A stop 34 may be provided to limit the movement of the disk.

Movement of valve disk 30 is controlled by a relatively large diameter (0.5 in.) permanently magnetic armature disk 35, preferably of samarium-cobalt. The armature magnet 35 is preferably sealed within a thin walled titanium shell forming part of the valve disk, as illustrated. Biasing is accomplished by means of further magnetic biasing means such as permanent smaller magnetic disks or ferro-magnetic shunt plates 36 and 37 to bias the valve disk in the desired position. The biasing magnets are preferably of samarium-cobalt having fields of intensity equal to or preferably less than the field of the armature magnet and with their magnetic axes in substantial alignment. The force of attraction between the armature magnet and the biasing magnets is proportional to the area of the pole divided by the distance to the third power. Accordingly, by adjustment of the distance of each magnet or shunt from the armature magnet, the armature can be made to latch in either open or closed state or can be set to give any ratio of opening to holding force when operated in a non-latching mode, as explained in greater detail herein after.

Bypass flow line 13 includes a capillary flow restrictor 38. Flow line 15, in series, includes a capillary flow restrictor 39 of lesser resistance. In the instance of insulin, the relative resistances of restrictors 38 and 39 may be in the ratio of 15 to 1. Thus, so long as the valve is closed, insulin bypassing the valve and subject to the greater resistance of restrictor 38 is infused at a low rate. When the valve is opened by magnetic action on the armature magnet 35 and the insulin flows through the valve through flow line 14 to the restrictor 39 of lesser resistance, then the drug is administered at a substantially higher rate.

The valve is adapted to be implanted within the body in close proximity to the skin surface with the magnetic axis of the armature magnet perpendicular to the body surface. The armature magnet reacts to the presence of another strong permanent magnet placed outside the body and aligned to the same axis, or to an electromagnet implanted adjacent to the valve body. The field can penetrate the body tissue and the housing of the control valve with no effect on either. If like poles are adjacent, the two magnets will repel each other. If unlike poles are adjacent, the two magnets will attract each other. The valve is preferably in a normally closed position. To prevent accidental opening of the valve by the presence of ferromagnetic material outside the body, the repulsion mode is selected to open the valve and the attraction mode is used to hold the valve shut. Common iron or steel objects are attracted by the internal magnet and only increase the force holding the valve shut. Strong magnetic fields of the intensity needed to open the valve are not common objects that would be encountered outside of specialized technical areas.

Where one biasing permanent magnet or ferromagnetic shunt is used, it is so positioned and spaced to maintain the valve disk in normally closed position assisted by the spring force of the valve disk and fluid pressure of the drug within chamber 26 on the disk. The activating outside permanent magnet or implanted electromagnet must be strong enough to overcome these combined forces. The valve will remain open so long as the outside permanent magnet is held in place or the electromagnetic coil is energized.

Where a pair of small permanent magnets or ferromagnetic shunt plates are disposed on opposite sides of the armature magnet, the valve disk may also be latched into open position. The net force on the armature magnet is the sum of the two opposing forces and the force between the armature and closest biasing magnet or shunt dominates. By adjustment of the distance of each magnet or shunt, the holding force in each direction can be set. These holding forces are set so that the stronger holding force maintains the valve in normally closed position. The valve now needs a large impulse of force to open, supplied by the external magnet or internal electromagnet. Once open, the much smaller holding force of the biasing means will keep it there.

Where the primary activation of the valve is from an internal implanted electromagnet, an external permanent magnet can be used to overpower the internal electromagnet to provide an override control function. If the external magnet is placed to pull on the armature, the electromagnet cannot exceed the combined pull of the internal bias magnet or shunt and the external control magnet. If the value is already open, the external magnet can exceed the holding force of the bias magnet or shunt (or the electromagnet) and force the value shut. If the electromagnet fails to operate to open the valve, the external magnet can be used to open the valve and hold it open as long as the magnet is held in place. If the electromagnet tries to impulse the valve shut, the external magnet will immediately reopen the valve or, if the magnet is strong enough, completely override the closing impulse.

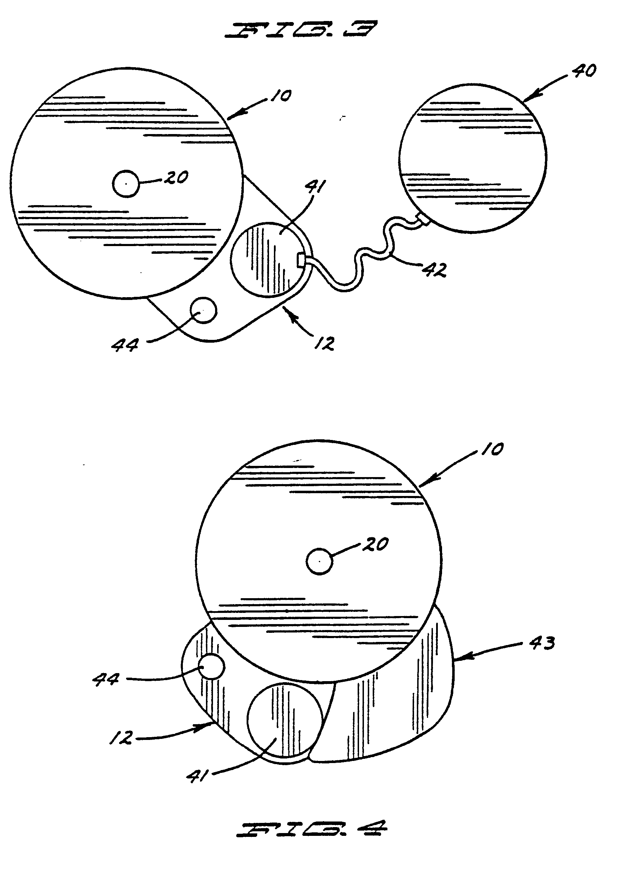

As shown schematically in Figure 2, the electronic package may contain a circuit to generate a series of impulses to hold the valve open in a programmed sequence of time intervals. A clock circuit may supply timing impulses. The timing impulses may be stored in semi-conductor or other electronic memory circuits. The control circuit puts out a voltage to the amplifier which drives the electromagnetic coil lcated in the proximity of the valve. The program can be altered by controlled impulses delivered to the implanted control circuit from an external transmitter. The electromagnet can serve as a telemetry antenna to pick up these control signals. The implanted electronic circuit may be as complex as needed to supply the correct delivery algorithm for the particular drug to be infused.

The electromagnetic coil is placed over the armature magnet outside the valve housing. The coil is wound as a flat spiral of several layers. The coil covers the region of high field intensity which is the size of the armature magnet. When current is sent through the coil, the magnetic field of the coil reacts with the field of the armature magnet to produce attraction or repulsion force, depending on the current flow direction. A larger current is needed to open the valve against the combined forces of the bias means, spring force and fluid pressure at the seal. Once open, a smaller current can hold the valve open. To insure prompt closing, a pulse of current in the reverse direction will provide the force to accelerate the armature to the closed condition. The coil is preferably enclosed in a thin titanium shell to reduce eddy currents from the changing magnetic field. The magnetic field penetrates the metal between the coil and the armature magnet with no losses other than from the spacing gap.

As seen in Figures 3 and 4, the control circuit may be housed in an electronic package 40 remote from the pump and valve and connected to the electromagnetic coil 41 through a flexible conductor conduit 42, or an electronic package 43 may be shaped to conform to and mate with the rest of the pump and the valve package and be held in place using medical grade silastic cement. The magnetic valve may be combined with other elements into a flow control package external to the pump which supplies a constant source of pressurized drug. That package may include the flow restrictors and a flow control regulator. An auxiliary septum 44 may be provided connected to the outlet to the catheter to allow direct injections into the infusion site, bypassing the controlled delivery system.

By physically separating the components of controlled drug delivery, each can be optimized for its function. The pump must store drugs for long periods of time and prevent degradation by chemical or bacterial action. The control unit must handle the drug safely but, due to the rapid throughput, avoidance of degradation is a lesser requirement. It has adjustable and calibrated parts that set up minimum and maximum flow rates. To accommodate a wide range of drug types, the control unit of the correct flow range is mated with the pump of the size needed. These components are heat sterilizable and care must be taken to eliminate any micro-organisms that could live in the drug stored in the pump. The electronics package must contain batteries and other heat sensitive components that cannot be heat sterilized. Since the electronics package is separate from the pump and control unit, it can be sterilized by chemical means. After each has been separately sterilized, the electronics package and pump control unit are mated at the time of implantation.

The specific embodiments described are given by way of example only (see Article 69 EPC).

**Claims**

1. An implantable magnetically controlled system for the infusion of drugs into an animal body from a pressure actuated drug delivery device (10), said system including a body (22, 23) of inert biocompatible nontoxic material and movable valving element (30, 32) located within said body in a cavity (26) having an inlet (28) leading into the cavity and an outlet (29) leading therefrom for connectiion to a catheter, characterized in that the cavity (26) is a shallow cavity and the movable valving element (30, 32) within the cavity (26) is positioned so as to move between an open position in which both the inlet (28) and the outlet (29) are free and a closed position in which one of the said inlet or outlet is obstructed, and has associated therewith a permanent armature magnet (35) located within the cavity and actuated by magnetic actuation means external to and separate from said body, and further characterised by the provision of biasing magnet means in the form of one or a pair of permanent magnets or ferromagnetic shunt plates (36, 37) within said body which, when the valving element (30, 32) is in its closed position, holds it in that position in the absence of any external magnetic field, said biasing magnet means being of equal or lesser field intensity compared to the strength of the armature magnet (35).

2. A system according to claim 1, wherein the said inlet (28) and outlet (29) lead into opposite sides of the cavity (26).

3. A system according to claim 1 or claim 2, wherein the valving element (30, 32) is a flap disk secured at one end to the body (22, 23).

4. A system according to claim 3, wherein the outlet (29) is disposed at one side of the cavity (26) underlying the disk and a resilient O-ring (31) is disposed around the outlet opening under the disk (30).

5. A system according to any preceding claim, including a bypass passage (27) extending from said inlet (28) to one end of a flow restrictor (38) of which the other end is connectable to the said catheter.

6. A system according to claim 5, wherein the bypass passage (27) extends through the body (22, 23).

7. A system according to claim 5 or claim 6, wherein a second flow restrictor (39) or lesser resistance than the said flow restrictor (38) is connected in series with the outlet (29).

8. A system according to any preceding claim, wherein said biasing magnet means consists of two bias magnets (36, 37) disposed so as to be closely spaced from opposite sides of the armature magnet (35) with magnetic axes aligned and unlike poles adjacent.

9. A system according to claim 8 wherein the two bias magnets (36, 37) provide open and closed latching positions of the valve.

10. A system according to any one of claims 1 to 7, wherein said biasing magnet means consists of one bias magnet (36 or 37) positioned relative the armature magnet (35) to normally maintain the valve in its closed position.

**Patentansprüche**

1. Implantierbares magnetgesteuertes System für die Infusion von Arzneimitteln bzw. Medikamenten in einen lebenden Körper bzw. den Körper eines menschlichen bzw. tierischen Lebewesens von einer druckbetätigten Arzneimittelabgabevorrichtung (10), welches System einen Körper (22,23) aus biologisch verträglichem, nichttoxischen Material und ein bewegbares Ventilelement (30, 32) aufweist, das innerhalb des Körpers in einem mit einem Einlaß (28) und einem mit einem Katheter verbindbaren Auslaß (29) versehenen Hohlraum (26) angeordnet ist, dadurch gekennzeichnet, daß der Hohlraum (26) ein seichter Holhraum ist und das bewegbare Ventilelement (30, 32) innerhalb des Hohlraumes (26) so angeordnet ist, daß es sich zwischen einer offenen Stellung, in welcher sowohl der Einlaß (28) als auch der Auslaß (29) freiliegen, und einer geschlossenen Stellung bewegt, in welcher entweder der Einlaß oder der Auslaß verschlossen ist, daß ihm ein Dauermagnetanker (35) innerhalb des hohlraumes zugeordnet ist, welcher von einer magnetischen Betätigungseinrichtung betätigbar ist, die außerhalb des Körpers und getrennt von diesem angeordnet ist, und daß weiters eine vorspannende Magneteinrichtung (36, 37) in der Form eines(r) oder eines Paares von Permanentmagneten oder ferromagnetischen Nebenschlußplatt(en) innerhalb des Körpers vorgesehen ist, die, wenn sich das Ventilelement (30, 32) in geschlossener Stellung befindet, dieses bei Fehlen eines äußeren Magnetfeldes in dieser Stellung hält, wobei die vorspannende Magneteinrichtung gleiche oder geringere Feldstärke im Vergleich zur Stärke des Magnetankers (35) besitzt.

2. System nach Anspruch 1, dadurch gekennzeichnet, daß der Einlaß (28) und der Auslaß (29) zu gegenüberliegenden Seiten des Hohlraumes (26) führen.

3. System nach Anspruch 1 oder 2, dadurch gekennzeichnet, daß das Ventilelement (30, 32) eine klappbare Scheibe ist, deren eines Ende am Körper (22, 23) befestigt ist.

4. System nach Anspruch 3, dadurch gekennzeichnet, daß der Auslaß (29) auf einer Seite des Hohlraumes (26) die Scheibe unterlagernd angeordnet ist und daß ein elastischer O-Ring (31) um die Auslaßöffnung herum angeordnet ist, die sich unterhalb der Scheibe (30) öffnet.

5. System nach einem der vorhergehenden Ansprüche, dadurch gekennzeichnet, daß ein Bypasskanal (27) vorgesehen ist, der sich vom Einlaß (28) zu einem Ende eines Strömungsdrosslers (38) hin erstreckt, dessen anderes Ende mit dem Katheter verbindbar ist.

6. System nach Anspruch 5, dadurch gekennzeichnet, daß sich der Bypasskanal (27) durch den Körper (22, 23) hindurch erstreckt.

7. System nach Anspruch 5 oder 6, dadurch gekennzeichnet, daß ein zweiter Strömungsdrossler (39) von geringerem Widerstand als der erste Strömungsdrossler (38) in Reihe mit dem Auslaß (29) geschaltet ist.

8. System nach einem der vorhergehenden Ansprüche, dadurch gekennzeichnet, daß die vorspannende Magneteinrichtung von zwei Vorspannmagneten (36, 37) gebildet ist, die in geringem Abstand auf gegenüberliegenden Seiten des Magnetankers (36) mit fluchtenden Magnetachsen und mit ungleichen Polen benachbart angeordnet sind.

9. System nach Anspruch 8, dadurch gekennzeichnet, daß die beiden Vorspannungsmagnete (36, 37) offene und geschlossene Verriegelungsstellungen des Ventils schaffen.

10. System nach einem der Ansprüche 1 bis 7, dadurch gekennzeichnet, daß die vorspannende Magneteinrichtung aus einem Vorspannmagnet (36 oder 37) besteht, der so in bezug auf den Magnetanker (35) angeordnet ist, daß er im Normalfall das Ventil in seiner geschlossenen Stellung hält.

**Revendications**

1. Système implantable à commande magnétique pour l'administration de médicaments par perfusion dans un corps vivant à partir d'un dispositif (10) de distribution de médicaments actionné par pression, ledit système comprenant un corps (22,23) en matière inerte biocompatible non toxique et un élément mobile d'obturation (30,32) logé à l'intérieur dudit corps dans une cavité (26) ayant une entrée (28) conduisant vers l'intérieur de la cavité et une sortie (29) qui en part pour être reliée à un cathéter, caractérisé en ce que la cavité (26) est une cavité peu profonde et en ce que l'élément mobile d'obturation (30,32) situé à l'intérieur de la cavité (26) est positionné de façon à se déplacer entre une position d'ouverture dans laquelle à la fois l'entrée (28) et le sortie (29) sont libres et une position de fermeture dans laquelle l'une desdites entrée ou sortie est obturée, un aimant permanent d'armature (35) étant associé à l'élément mobile d'obturation, disposé à l'intérieur de la cavité et actionné par des moyens magnétiques d'actionnement extérieurs et séparés dudit corps, et en outre caractérisé par la présence de moyens magnétiques (36,37) de poussée, sous la forme d'un ou deux aimants permanents ou plaquettes formant shunts ferromagnétiques, disposés à l'intérieur dudit corps qui, lorsque l'élément d'obturation (30,32) est dans sa position de fermeture, le maintiennent dans cette position en l'absence de tout champ magnétique extérieur, lesdits moyens magnétiques de poussée ayant une intensité de champ égale ou inférieure, comparée à la force de l'aimant d'armature (35).

2. Système selon la revendication 1, dans lequel lesdites entrée (28) et sortie (29) conduisent dans des côtés opposés de la cavité (26).

3. Système selon la revendication 1 ou la revendication 2, dans lequel l'élément d'obturation (30,32) est un disque plat fixé par une première extrémité au corps (22,23).

4. Système selon la revendication 3, dans lequel la sortie (29) est disposée sur un premier côté de la cavité (26) au-dessous du disque et une bague torique élastique (31) est disposée autour de l'ouverture de sortie au-dessous du disque (30).

5. Système selon l'une quelconque des revendications précédentes, comprenant un passage de dérivation (27) s'étendant de ladite entrée (28) jusqu'a une première extrémité d'un organe d'étranglement d'écoulement (38) dont l'autre extrémité peut être reliée au cathéter.

6. Système selon la revendication 5, dans lequel le passage de dérivation (27) s'étend à travers le corps (22,23).

7. Système selon la revendication 5 ou la revendication 6, dans lequel un second organe d'étranglement d'écoulement (39) de plus faible résistance que ledit organe d'étranglement d'écoulement (38) est relié en série à la sortie (29).

8. Système selon l'une quelconque des revendications précédentes, dans lequel lesdits moyens à aimants de poussée comprennent deux aimants de poussée (36,37) disposés de façon à être faiblement espacés de côtés opposés de l'aimant d'armature (35), les axes magnétiques étant alignés et des pôles différents étant adjacents.

9. Système selon la revendication 8, dans lequel les deux aimants (36,37) de poussée établissent des positions de verrouillage en ouverture et en fermeture de la valve.

10. Système selon l'une quelconque des revendications 1 à 7, dans lequel lesdits moyens à aimants de poussée comprennent un aimant de poussée (36 ou 37) positioneé, par rapport à l'aimant d'armature (35), afin de maintenir normalement la valve dans sa position de fermeture.

TO CATHETER

*FIG. 1*

MEMORY CIRCUITS

CLOCK

CONTROL CIRCUIT

AMPLIFIER

COIL

TELEMETRY ANTENNA

*FIG. 2*

1

EP 0 031 850 B2

*FIG. 3*

*FIG. 4*